(19)

Europäisches Patentamt
European Patent Office
Office européen des brevets

(11) **EP 2 849 731 B1**

(12) **EUROPEAN PATENT SPECIFICATION**

(45) Date of publication and mention of the grant of the patent:
**04.01.2017 Bulletin 2017/01**

(21) Application number: **13723129.6**

(22) Date of filing: **15.05.2013**

(51) Int Cl.:
*A61K 9/20* (2006.01)    *A61K 9/28* (2006.01)
*A61K 36/9066* (2006.01)

(86) International application number:
**PCT/EP2013/060059**

(87) International publication number:
**WO 2013/171270 (21.11.2013 Gazette 2013/47)**

(54) **FORMULATION FOR LOW BIOAVAILABILITY SUBSTANCES CONTAINING A N-ACETYLCYSTEINE-CHITOSAN SALT**

FORMULIERUNG MIT N-ACETYLCYSTEIN-CHITOSANSALZ FÜR WITKSTOFFE MIT GERINGER BIOVERFÜGBARKEIT

FORMULATION CONTENANT UN SEL DE N-ACÉTYLCYSTÉINE-CHITOSANE POUR SUBSTANCES DE FAIBLE BIODISPONIBILITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2012 IT MI20120847**

(43) Date of publication of application:
**25.03.2015 Bulletin 2015/13**

(73) Proprietor: **Labomar S.r.l.**
**31036 Istrana (TV) (IT)**

(72) Inventor: **FRATTER, Andrea**
**I-31036 Istrana (TV) (IT)**

(74) Representative: **Asensio, Raffaella Consuelo et al**
**Perani & Partners S.p.A.**
**Piazza San Babila, 5**
**20122 Milano (IT)**

(56) References cited:
**EP-A1- 2 149 377        KR-B1- 100 750 727**
**US-A1- 2010 239 663**

• **HOMBACH ET AL: "Thiolated chitosans: Development and in vitro evaluation of an oral tobramycin sulphate delivery system", EUROPEAN JOURNAL OF PHARMACEUTICAL SCIENCES, ELSEVIER, AMSTERDAM, NL, vol. 33, no. 1, 11 December 2007 (2007-12-11), pages 1-8, XP022384905, ISSN: 0928-0987**

Remarks:
The file contains technical information submitted after the application was filed and not included in this specification

EP 2 849 731 B1

**Description**

FIELD OF THE INVENTION

The present invention relates to a composition comprising: a core comprising at least one active principle with low bioavailability, a polysorbate and a N-acetylcysteine-chitosan salt, wherein this core is enclosed in an enteric coating layer, to a method for its production, to said composition for use as a medicament, to a food supplement or a nutraceutical composition comprising said composition.

A large number of substances that are used in pharmaceutical formulations and/or in nutraceutical and food supplements show a reduced or poor bioavailability, due to the failure of enteral absorption and metabolization, or to chemical denaturation that may occur in the gastro-intestinal lumen as in hepatic cytochromes. Because of these physiological phenomena, food supplements and pharmaceutical and nutraceutical formulations are often totally ineffective or markedly less effective than expected on the basis of the in vitro data. General and non-limiting examples of such substances with low bioavailability are vitamins, polyphenols, terpenes steroidal, non-steroidal terpenes, fatty acids and coenzymes.

The absorption of hydrophilic substances through the enteric mucosa is severely limited by biophysical and biochemical factors that result in reduced bioavailability.

Weakly acidic, lipophilic and low molecular weight (<1000 Daltons) substances (e.g. acetylsalicylic acid) are absorbed through the gastric mucosa via active trans-cellular transport mechanisms or via facilitated diffusion. Instead, the weakly basic substances are ionized in the stomach where the pH is about 1.5, therefore they are not able to be absorbed through the gastric mucosa. Such mildly alkaline substances reach the ileum and the duodenum where they are absorbed by virtue of the large surface contact area with the intestinal mucosa and of the higher permeability of cell membranes. The substances with marked lipophilic character, such as fat-soluble vitamins (A, D, E, K, F), are assimilated only after their emulsification by bile salts, which allows the formation of micelles that facilitate the contact between the lipophilic substances and the surface of the intestinal villi. In the gastro-enteric mucosa area, the main mechanisms that slow down or disrupt the trans-wall penetration of substances introduced via the oral route are:

- the thickness of the mucous secretions, that can trap the active principle and make it unavailable to penetration;
- the metabolism by CYP3A enzyme (present in the villous enterocytes);
- the extrusion by P-glycoprotein (present in the villous enterocytes);
- the peptidic nature of the substances (proteolytic gastric-acid hydrolysis);
- the reduced trans-mural flow due to enteric tight junctions.

In fact, the presence of the anatomical barrier represented by "tight junctions" is extremely important to understand the mechanisms of enteral absorption of active principles. Tight junctions are plaque protein structures that maintain the contact between epithelial cells, favouring an anatomical continuity of the epithelium and a reduced trans-mural flow of exogenous substances through it.

One possibility for the administration to a subject of substances that are poorly absorbed in the intestine is to formulate them using nanoemulsions (e.g. Patent Application IT2008VE00055) or for trans-lingual absorption (e.g. patent application WO2011161501).

However, for some substances and some therapeutic indications, it would be preferable to achieve enteric release and absorption.

The substances of particular interest are curcumin, a polyphenolic compound extract from the root of *Curcuma longa,* and berberin, an alkaloid extracted from *Berberis* genus plants with an isoquinoline ring-system, that have demonstrated interesting and well-documented immuno-modulating and anti-inflammatory properties in the treatment of inflammatory autoimmune diseases, such as arthritis and colitis.

These substances, in spite of their unequivocal pharmacological and therapeutic potentials, are now of limited clinical applicability, because, in *in vitro* (CACO-2) and *in vivo* (on healthy human volunteers) studies, they show a very low bioavailability after oral intake, resulting in very low plasma peak and low Cmax and AUC.

The main common reasons for the very low bioavailability of both curcumin and berberin are due at least to the following:

- extrusion of the substance by the action of the enterocyte p-gp pump;
- biotransformation of the substances by the enterocyte cytochromes (CYP3A);
- reduced trans-mural flow related to the "tight junctions".

KR100750727 discloses formulations comprising a binary mixture of Chitosan and N-acetylcysteine, produced by condensation of N-acetylcysteine with chitosan by formation of covalent bonds. Said binary mixture demonstrates excellent dissolution properties, as well as the ability to reduce the effectiveness of active oxygen species and to absorb lipids from the intestine, reducing their systemic absorption, thus decreasing the concentration of cholesterol in the blood.

One purpose of the present invention is to provide a composition for oral intake that allows the release and absorption in the enteric tract of substances normally with little or no bioavailability, in particular of curcumin and berberin.

An object of the present invention is to provide a composition that allows the absorption of substances in the enteric level, by modulating the main biophysical and biochemical mechanisms that degrade these substances, or which prevent their crossing of the intestinal epithelium.

Another task of the present invention is to provide a process for the production of a pharmaceutical or nutraceutical formulation, or of a dietary supplement, that would allow the release and absorption in the enteric tract of substances, which normally have little or no bioavailability.

In accordance with the present invention, these purposes, and others that will be more apparent hereinafter, are achieved by a composition comprising:

a) a core comprising at least one active principle with low bioavailability, a polysorbate and a N-acetylcysteine-chitosan salt and

b) an enteric coating layer enclosing the core a) wherein said N-acetylcysteine-chitosan salt comprised in a) is obtained by the protonation of the free amino group of chitosan by means of N-acetylcysteine, behaving as an acid.

These objects have also been achieved through said composition for use as a medicament. In accordance with the present invention, these purposes have also been achieved through a dietary supplement or nutraceutical composition comprising said composition.

These aims were also achieved by a method for the formation of said composition comprising the steps of:

i. preparing a solution or an aqueous dispersion comprising at least a polysorbate in a concentration between 1 and 30% by weight/total weight of the solution and a N-acetylcysteine-chitosan salt wherein N-acetyl cysteine and chitosan are in a concentration between 0.01 and 3% by weight/total weight of the solution;

ii. wet-granulating a composition comprising at least the active principle with low bioavailability with the solution or aqueous dispersion obtained in step i.;

iii. drying to constant weight and sieving the granulate obtained in step ii.;

iv. forming the core a) of the composition by compression of the solid obtained in step iii.; and

v. coating the core a) obtained in step iv. with a gastro-resistant enteric layer.

In another aspect, herewith disclosed is a salt of chitosan salified with N-acetylcysteine, i.e. a N-acetylcysteine-chitosan salt.

In the context of the present invention, low bioavailability means a bioavailability of less than 30%, or 20%, or 10% or 5%.

In the context of the present invention, the term chitosan means a polysaccharide of natural or semisynthetic origin, and its ionized forms, with molecular weight greater than 5000 D (Dalton or molecular mass units) and a degree of deacetylation greater than 90%. Preferably, the molecular weight of chitosano in the composition of the present invention is 100000 Dalton, or higher.

In the context of the present invention, the term "essential oils" refers to products obtained by extraction of the "essences" from suitable plant material.

As used herein, the term "enteric coating layer" means a layer, forming a continuous barrier, that consists of or is coated with substances that are resistant to the acidic gastric juices and dissolve in the upper intestinal tract. Said enteric coating layer may be applied directly on the core (e.g. to obtain a filmed tablet or granule) or may be in the form of a shell (e.g. an enteric-coated hard or soft capsule shell comprising the core a), which encloses said core in the form of powder or of one or more tablets, granules or micropellets.

In the context of the present invention, "enteric film" means a film that is applied directly on the core, i.e. it adheres to the core, and is capable of protecting the composition from the action of the gastric juices in the stomach and to release the active principle in the upper intestine.

In the context of the present invention, "titrated extract" means an extract of a plant containing a standardized amount of a particular substance, measured via one of the standard analytical methods known to the person skilled in the art (e.g. referring to the US, UK or European Pharmacopoeias).

Unless otherwise specified, in the context of the present invention, percentages of the components of a mixture refer to weight of the component over the whole weight of the mixture (w/w).

As used herein, the term "comprises", referred to a component of a composition, is understood to encompass also the possibility that said component constitutes 100% of the composition, i.e. a mixture "comprising" component A may also consist of 100% of A.

In one aspect, the present invention provides a composition comprising:

a) a core comprising at least one active principle with low bioavailability, a polysorbate and a N-acetylcysteine-chitosan salt;

b) an enteric coating layer enclosing the core a) wherein said N-acetylcysteine-chitosan salt comprised in a) is obtained by the protonation of the free amino group of chitosan by means of N-acetylcysteine, behaving as an acid.

It was surprisingly found that the composition according to the invention allows the release and absorption by the intestinal walls of normally poorly bioavailable active principles.

Without intending to be bound by theory, it is believed that the advantages of the composition of the present invention are, at least partially, related to the presence of the salt of chitosan, which itself exerts a modulation of enteric epithelium tight junctions, with a substance which has a mucolytic action, i.e. N-acetylcysteine (NAC).

Chitosan is a polysaccharide composed of repeating units of N-acetylglucosamine and D-glucosamine. Chitosan can form aqueous macrocolloidal gels after acidification. Such gelation occurs after the protonation of the free amino groups of the N-acetylglucosamine, which follows the formation of a ammonium polycation salt. Precisely this process of polysalification determines the water solubility of the chitosan and its relative ability to gel.

NAC, behaving as an acid, protonates the free amino group of chitosan forming the salt. As used herewith, the term "N-acetylcystein-chitosan salt" refers to the product of this salt-forming acid-base reaction. This process involves only the formation of ionic bonds (i.e. between a positively and a negatively charged species), in the absence of covalent bonds. It was found that the ionized form of chitosan exerts an interaction with the tight junctions that is much higher than that exerted by the non-ionic form.

The thiolated or conjugated chitosan, defined as molecules produced by condensation of NAC with chitosan by formation of covalent bonds (promoted by condensation agents such as the carbodiimides) have already been disclosed in the prior art as intestinal absorption enhancers (Schmitz, T. et al. Drug Deliv. 2008, 15, 245; Chen, H. et al J. Liposome Res. 2011, epub ahead of print; Sakloesakun, D. et al. Drug Dev. Ind. Pharm. 2011, 37, 648; Schmitz, T . et al. Int. J. Pharm. 2008, 347, 79).

The ionic salt in the composition of the invention is different from the compounds disclosed in the prior art, e.g. deriving from the covalent attachment of NAC to chitosan, by formation of amide bonds between the primary amino group of the polymer and the carboxylic acid group of NAC (Hombach, J. et al. Eur. J. Pharm. Sci. 2008, 33, 1-8).

One of the advantages of the salt in the compositions of the present invention consists in providing chitosan in the cationic form, which is water-soluble and can act as a mucolytic agent on intestinal secretion, with superior efficacy compared to the conjugate compounds (with covalent bonds between NAC and chitosan) of the prior art.

In the N-acetylcysteine-chitosan salt in the composition according to the present invention, the weight ratio of chitosan and NAC may be from 1:1 to 3:1, in particular 2 to 1. Preferably, in the N-acetylcysteine-chitosan salt in the composition of the invention, N-acetylcysteine is in an amount such that an aqueous solution obtained dissolving said salt in water and containing 1% w/w chitosan has a pH between 3.5 and 4.0.

The presence of at least one polysorbate further facilitates the penetration into the circulation of substances taken orally. The polysorbate surfactants are oily liquids, derived from PEG-ylated sorbitan (a derivative of sorbitol) by esterification with fatty acids, which are widely used in the formulation of oil/water emulsion, of surfactant-based detergents and of topical forms. The polysorbates act as absorption promoters exerting an action of micelle formation of the water insoluble or poorly soluble active ingredients and acting as "cellular surfactants", actually altering the permeability of the membrane. In the composition of the present invention, the polysorbates thus contribute to making the active ingredient more freely available to intestinal absorption.

As non-limiting examples, the composition of the present invention can be in one of the following forms: a filmed tablet, a filmed granule, a capsule comprising enteric-filmed granules or micropellets, or a hard or soft capsule with a gastro-resistant coating comprising the core a) in the form of powder, granules, micropellets, solution or suspension.

Preferably, in the composition according to the invention the layer b) is an enteric film directly coating said core a), i.e. a film enclosing the core and applied in direct contact with it.

Preferably, in the composition according to the invention, the at least one active principle with low availability is selected from trans-resveratrol, melatonin, omega-3 fatty acids, steroidal terpenes as boswellic acids, non-steroidal terpenes, saponins, essential oils, polyphenols such as curcumin, berberin and mixtures thereof, peptides and oligopeptides such as glutathione. More preferably, in the composition according to the invention at least one active principle with low availability is curcumin or berberin. The content of active ingredient in the composition in the form of a tablet according to the invention can be between 0.01 and 1000.0 mg, preferably between 50 and 500 mg, more preferably between 100 and 300 mg per tablet.

The polysorbate included in the core a) of the composition of the present invention may be one of the commercially available polysorbates, such as polysorbate 20, 40, 60, 65, 80 and mixtures thereof, in amount between 20 and 300 mg per single tablet. Preferably, in the composition according to the invention, the polysorbate is polysorbate 80.

Preferably, the core a) of the composition according to the invention also comprises an extract of *Citrus x paradisi* titrated to at least 40% by weight/total weight of the extract of *Citrus x paradisi* in bioflavonoids, and specifically titrated in Naringin and Naringenin, and a *Piper nigrum* extract titrated to 95% by weight/total weight of the extract of *Piper nigrum* in piperine.

The extracts of grapefruit and black pepper modulate the activity of several enzymes responsible for the biotransformation of xenobiotics and in particular of the hepatic cytochrome P450 and the type and intestinal CYP3A.

These extracts can be in the form of phytocomplexes, i.e. of a mixture comprising all the substances extractable from the plant that are endowed with and that contribute to achieving a specific biological activity.

Indeed, it is known that some substances are able to interact with the P-gp pump located in the outer membrane of the enterocyte inhibiting it. This effect results in an increased passage in the entero-portal circulation and in a decreased metabolism by intestinal and hepatic cytochrome, which leads to a greater effectiveness of the orally taken active ingredients. This mechanism of action is effected in particular by some flavonoids in the Citrus genus.

Such flavonoid complexes are also able to inhibit the CYP-3A enzyme of the enterocytes, and in some cases also that of the liver, thereby reducing the pre-systemic biotransformation.

It was found that, also through this mechanism, with the compositions according to the present invention it is possible to induce an increase in plasma concentrations of substances that would otherwise be massively biotrasformated yet before entry into the circulation.

The core a) of the composition according to the present invention is subjected to a process of filming, for example in a coating pan, with a dispersion of a substance capable of forming a film resistant to digestive juices, to obtain the gastro-protection. The active substances and those intended to act as enteric absorption promoters are actually released only in the enteric and gastric traits. The early release of chitosan in the gastric environment, in fact, would cause the polymer to swell, due to the highly acidic environment for hydrochloric acid, and induce the formation of a gel. The active principles and other substances might thus be released in the stomach and be degraded. Preferably, the enteric coating (b) comprises at least one or a combination of derivatives of methacrylic acid and methyl methacrylate (as non-limiting examples: Eudragit™ L100, Eudragit™ L12,5, Eudragit™ S100, Eudragit™ S12,5), shellac (Shellac™) or methyl cellulose and its derivatives. Preferably, the coating b) comprises or consists of shellac (Shellac™). Such a coating can be achieved by filming the core a) with a dispersion of shellac in water.

Preferably, the composition according to the invention is in the form of an enteric granule, wherein the core a) is enclosed in the enteric film b), or of a gastro-resistant capsule wherein said core a) is enclosed in a hard or soft shell (layer b)that is enterically coated).

In another preferred embodiment, the composition of the present invention is in tablet form and comprises a layer c), outer to the coating b), comprising at least one excipient suitable for compression. Preferably, said excipient of the outer layer is selected from starch, microcrystalline cellulose, magnesium stearate, calcium phosphate, talc, silica and mixtures thereof.

In the composition according to the present invention, the core a), the coating b) and the outer layer c) may comprise further excipients and/or active ingredients in addition to those listed above.

Non-limiting examples of additional ingredients are: anhydrous dibasic calcium phosphate, colloidal silica, amorphous silica, sorbitol and starch.

In another embodiment, the present invention provides said composition comprising the core a) and the coating b) as described above for use as a medicament.

In another embodiment, the present invention provides a dietary supplement or a nutraceutical composition comprising the composition comprising the core a) and the coating b) as described above.

The administration of said composition or dietary supplement to a subject advantageously results in a degree of enteric absorption of poorly bioavailable substances that is higher compared to that achieved via the formulations of the prior art.

In another embodiment, the present invention provides a method for the preparation of said composition comprising the steps of:

i. preparing a solution or aqueous dispersion comprising at least a polysorbate in a concentration between 1 and 30% by weight/total weight of the solution and a N-acetylcysteine-chitosan salt, wherein N-acetyl cysteine and chitosan are in a concentration between 0.01 and 3% by weight/total weight of the solution;
ii. wet-granulating a composition comprising at least an active principle with low bioavailability with the solution or aqueous dispersion obtained in step i.;
iii. drying to constant weight and sieving the granulate obtained in step ii.;
iv. forming the core a) of the composition by compression of the solid obtained in step iii.;
v. coating of the core a) obtained in step iv. with a gastro-resistant enteric layer.

In the N-acetylcysteine-chitosan salt of step i., the amount of N-acetylcysteine is such that an aqueous solution obtained dissolving said salt in water and containing 1% w/w chitosan has a pH between 3.5 and 4.0.

Preferably, in the method according to the invention, the granulation of step ii. is performed by combining the solution or aqueous dispersion obtained in step i. and a composition comprising the active substance with low bioavailability, an extract of *Citrus x paradisi* titrated to at least 40% by weight/total weight of the extract of *Citrus x paradisi* in bioflavonoids, and specifically titrated in Naringin and Naringenin, and/of an extract of *Piper nigrum* titrated to 95% piperine by weight/total weight of the extract of *Piper nigrum* and, if necessary, N-acetylcysteine and chitosan in such quantities as to obtain in a single dosage unit an amount of chitosan between 1 and 150 mg, an amount of N-acetylcysteine between 1 and 75 mg, an amount of extract of grapefruit (*Citrus x paradisi*) between 1 and 300 mg, and a quantity of extract of *Piper nigrum* between 1 and 30 mg.

Preferably, said composition comprising the low bioavailability active, extracts of grapefruit and pepper and optionally chitosan and N-acetylcysteine further comprises at least one excipient for pharmaceutical, nutraceutical and/or food products.

The coating of the core a) of step iv. of the method according to the present invention can be made, for example, in a coating pan with a solution or dispersion in water of an agent capable of forming an enteric film, preferably with a 25% w/w aqueous dispersion of shellac (SHELLAC™).

Preferably, in the method of the invention, the wet granulation step ii. is carried out via a fluid bed process.

The composition in the form of granules can be directly obtained by a method comprising the steps i.-v. as described above.

Preferably, for the formation of the composition in tablet form, the method according to the invention further comprises:

vi. adding to the filmed core obtained in step v. at least one excipient suitable for tabletting;
vii. tabletting the composition obtained in step vi. to obtain the composition in the form of tablet.

The following examples are provided to describe particular embodiments of the present invention, without intending to limit its scope.

Example 1

Preparation of the granulate containing the active ingredient.

Active ingredient:

*Curcuma Longa* (Dry Extract, 95% w/w Curcumin) 200 mg per tablet

Step 1

Tablet core of granular:

A mixture of dry powders is prepared containing:

*Curcuma Longa* dry extract (95% w/w curcumin) 100 g.
Chitosan HD 100 Mesh 75.0 g
N-acetylcysteine 25.0 g
Grapefruit Seed dry extract (50% w/w bioflavonoids) 25.0 g (optional).
Black pepper fruit Dry Extract (95% w/w Piperine) 2.50 g (optional).
Corn starch 75.0 g white.
Tot 302.5 g.

Step 2

The granulating solution is obtained as follows:

Deionized water for reverse osmosis (120 ml) is introduced into a 250 ml conical flask and 2 grams of chitosan (MW≥ 5000 D, degree of deacetylation> 90%) are dispersed under stirring with a magnetic plate; 1.0 grams of NAC are added so as to obtain a pH between 3.5 and 4.0, measured with a pH meter, simultaneous checking the solubilization of chitosan and the concomitant formation of a gel. To the solution thus obtained 29.0 grams of polysorbate 80 are combined until complete dispersion, obtaining a light yellow-coloured translucent system. Deionized water (obtained via reverse osmosis) is then added to obtain a final volume of 200 ml.

Step 3

Granulation

The mixture of powders obtained (302.5 g) in step 1 is placed in a plastic beaker and the granulating solution obtained in Step 2 is added under constant mechanical stirring in subsequent portions, checking the progressive triggering of the granulation process (total granulating solution added to the powder mixture: 140 g). After the granulated core is obtained, it is dried in a ventilated oven at 50°C until constant weight (anhydrification). The dried granulate is then sieved. Fine granules are finally obtained with a weight of 414.5 gr.

Step 4

Obtaining tablets

The thus obtained granules (248.7 g) are then added with microcrystalline cellulose (12.3 g), calcium hydrogen phosphate anhydrous (Fujicalin) (60.0 g), microcrystalline cellulose (CEOLUS KG802) (30.0 g), magnesium stearate (5.4 g) and talc (3.6 g). The powder mixture thus obtained is mixed in a suitable laboratory V mixer for 2 minutes. The thus obtained powder (359.3 g) is compressed into tablets, which are coated with a 25% w/w solution of shellac (ShellaC ™) in coating pan for a total of 0.06623 g to obtain 300 tablets with final net weight equal to 360.0 g (300 tablets).

EXAMPLE 2

1. Introduction and objective

Intestinal absorption is a complex process wherein two types of transport can be distinguished: the so-called passive transcellular and paracellular transport and the active transport or facilitated diffusion, mediated by specific receptors.
The process can be influenced by the intestinal metabolism, by the action of efflux transporters as well as by some characteristics of the chemical compound that must be absorbed, including the molecular weight, the size and conformation of the molecule, the degree of ionization, the lipophilicity, solubility and dissolution in gastric contents. To predict the intestinal absorption and the transport through the mucosa, a model for in vitro study is proposed using the Caco-2 cell monolayer, which protocols are being validated as an alternative to animal testing at ECVAM (Le Ferrec E. ey al., 2001 ECVAM workshop 46).
The good in vivo / in vitro correlation for the passive transport of drugs characterizes the relevance and dissemination of the Caco-2 model in the study of intestinal absorption.
The Caco-2 line is widely used as an in vitro model of intestinal epithelium for barrier toxicity studies and for qualitative screening of intestinal absorption, especially in the pharmaceutical industry during the stages of research and development for the identification and optimization of lead compounds (Garate M.A. et al.,J. Biol. Chem. 2000, 275, 1671; Artursson, P, and Borchardt R.T. "Intestinal drug adsorption and metabolism in cell cultures: Caco-2 and beyond". Pharma. Res. 1997, 14, 1655-1658). The cells are grown on specific porous supports housed in wells for cell cultures, that allow to have two distinct sectors: an apical sector, corresponding to the intestinal lumen in vivo, and a basolateral sector corresponding to the segment of the blood and lymphatic circulation of the organism.
The differentiation is carried out in 21 days: the Caco-2 differentiated cells at this time form a continuous epithelium with functional tight junctions and microvilli.
Absorption test on Caco-2 model is used for the evaluation of the passive intestinal passage of the core composition according to the invention in the form of a Curcuma-comprising granulate dispersed in water (hereafter: Composition A) at doses which are defined on the basis of a preliminary cyto-toxicity test.

2. Experimental design

Before testing the rate of permeability of the test substance, cytotoxicity on Caco-2 monolayer is evaluated by MTT test (in accordance with the internal procedures, 2 tests performed after respectively 2h and 1h of treatment) at different concentrations in triplicate for the definition of the non-cytotoxic dose:

1) FIRST ROUND: 0.25% /0.5% /1%> 2 h of treatment
2) SECOND ROUND: 0.025% /0.05% / 0.1% /0.25%> 1 h of treatment.

The chosen concentration for the intestinal absorption of curcumin is 0.25% w/w of granulate containing 0.052% w/w of curcumin (1.404 mM), with 1 hour of treatment. The curcuma-containing (non-filmed) granulate of the composition according to the present invention (as obtained in step 3 of Example 1, hereafter indicated as Composition A) is tested for the intestinal passage, after dissolution/suspension in 1% HBSS-MES buffer (buffer at pH 6.5 used in the apical part).

The passage through a monolayer of Caco-2 cells of Composition A at non-cytotoxic concentration is monitored at defined times (0 min- 15 min -30 min and 60 min). The liquid in the basolateral compartment is collected and the active substance of interest (curcumin) is quantified by HPLC.

The following parameters are evaluated to verify the integrity of the monolayer and to measure the permeability changes that occur after intestinal passage:

- Integrity test of barrier, by measuring the transepithelial resistance (TEER);
- Paracellular passage with evaluation of the toxicity of the test substance on the integrity of the barrier function by the use of a marker of paracellular passage (Lucifer yellow) using the highest non-cytotoxic concentration according to the results of the MTT assay.

3. Materials

3.1. Test system

3.1.1 CACO-2 Cell line

The Caco-2 line consists of cells derived from intestinal cells (human colorectal adenocarcinoma) provided by ATCC.

Caco-2 cells grow in monolayer in DMEM medium containing 4.5 g / L of glucose with the addition of 10% FBS, 1% nonessential amino acids, 4 mM glutamine, 10 mM Hepes and antibiotics (Pen / Strep).

The cells reach confluence in about 6 days and reach a stationary stage in 10 days. The differentiation is complete in about 21 days. The differentiated cells have high levels of alkaline phosphatase, aminopeptidase and isomaltase.

The structural and functional differentiation of the microvilli is associated with the polarization of the monolayer after confluence. The polarity is also evident for the presence of tight junctions that form during the differentiation.

The integrity of the monolayer is confirmed by the low permeability of the monolayer to mannitol or lucifer yellow (Hidalgo et. al. "Characterization of the human colon carcinoma cell line (Caco-2) as a model for intestinal epithelial permeability" Gastroenterology 1989, 96, 736-749 and Le Ferrec E. "In vitro models of the intestinal barrier" ECVAM workshop 46, ATLA 2001, 29,649-668).

3.1.2 Culture conditions and use of the test system

The cells (150.000/insert, counted using Buerker chamber) are plated in inserts with PET filter (Millipore, 10mm in diameter with a pore size of 0.4 mm) for 12 wells plates and are incubated at 37°C with 5% $CO_2$ and grown for 21 days. Additional 0.5 ml of medium are added over the insert and 1.5 ml of medium are added in the plate.

The medium is changed every second day. From day 21 the morphological analysis reveals a complete differentiation of Caco-2 cells.

3.2.1 Instrumentation

Analytical Scale XS 204(Mettler- Toledo)
$CO_2$ Incubator Hera Cell (Heraeus)
Microplate Autoreader M200-Infinite (Tecan)
ERS-Millicell (Milipore)
DM-IL microscope (Leica)
Buerker Chamber (Marienfeld)
3.2.2 Reagents
DMEM 12-614F LONZA
GLUTAMINE BE17-605E LONZA
PEN/STREP DE17-602E LONZA
NEAA SOLUTION 100X BE13-114E LONZA
FBS DE14-701F LONZA

HBSS BE10-527F LONZA
HEPES 1M 17-737F LONZA
MES M2933 SIGMA
LUCIFER YELLOW L0144 SIGMA
HBSS - 1% MES APICAL solution (Prepared in lab and used within a week)
HBSS - 1% HEPES BASOLATERAL solution (Prepared in lab and used within a week)

4. Methods

The tests are run in a GLP-certified test facility.

4.1 MTT assay: Cytotoxicity of the test substances on the Caco-2 line

4.1.1 Principle of the method

The potential cytotoxic effect of the substances is evaluated on Caco-2 cells in proliferative phase using the MTT assay.
The MTT assay is considered an international standard for the quantification of cytotoxicity (ISO 10993-5). The method allows the quantification of a cytotoxic effect induced by the product through the measurement of cell viability. Cytotoxicity is quantified by measuring the decrease in cell viability compared to an untreated negative control. It is based on the metabolic reaction that occurs between the tetrazolium salt (MTT) and mitochondrial enzymes (succinate dehydrogenase) after contact with the product for different treatment times: only the living cell can transform the tetrazolium salt into the insoluble derivative (formazan). The reaction is revealed by the formation of a purple colouring at the insert base.
The purple-coloured derivative is extracted into isopropanol and the quantification is carried out via a spectrophotometry at 570 nm.

4.1.2 Procedure

The following concentrations are assessed:

3) 0.25% / 0.5% / 1% (FIRST ROUND MTT): 2h treatment
4) 0.025% / 0.05% / 0.1% / 0.25% (SECOND TEST MTT): 1h treatment The Caco-2 cells are plated in 96-well multiwall plate (in triplicate) at cell density of 120,000 cells per well (cell counting chamber by Buerker).

After incubation for 1h or 2h with the substance to be tested, the content in formazan for each well is assessed via spectrophotometry at 570 nm wavelength ($\lambda$).
After performing the subtraction of the blank, the % of vitality is obtained with respect to the untreated control according to the formula:

$$\text{VITALITY\%} = (\text{O.D. TREATED / OR UNTREATED CONTROL}) * 100$$

4.2 Assay for trans-epithelial electrical resistance (TEER): verification of the barrier function integrity.

4.2.1 Principle of the method

The trans-epithelial electrical resistance (TEER) is a direct measure of the skin barrier functionality: it reflects the overall strength of the tissue due both to its thickness and to its structure. TEER measures the integrity of the barrier at the level of tight junctions.
The value of trans-epithelial electrical resistance (TEER) of the monolayer, consisting of differentiated Caco-2 cells, represents an indication of the level of integrity of the monolayer, hence of the same formation of the tight junctions between the enterocytes. The measurement of the trans-epithelial resistance value (expressed in $\Omega * cm2$) is performed using a volt-ohmmeter (Millicell ERS) (range 0-2000 $\Omega$). The values of TEER of the differentiated monolayer vary in function of the cell line and are acceptable above 150-200 $\Omega* cm^2$.

4.2.2 Procedure

On a differentiated monolayer of Caco-2 cells, after 21 days of culture, TEER is evaluated by performing an insert measurement through a special electrode, placing the short end of the electrode within the insert in 0.5 mL medium. The long part of the electrode is immersed in 1.5 mL of medium in the well containing the insert.

4.3 Assay with Lucifer yellow: verifying the integrity of cell junctions in presence of the substance to be evaluated.

4.3.1 Principle of the method

Lucifer yellow is a fluorescent marker impermeable to the cell membrane.

Is used to study the permeability of a substance at paracellular level.

When the joints are undamaged, the lucifer yellow has a very low permeability.

4.3.2 Procedure

The transport experiments are performed on monolayer of Caco-2 cells applying at the level of the apical compartment of the insert 100 $\mu$mol / L of Lucifer yellow (LY) together with the test substance (the highest non-cytotoxic in the MTT test), dissolved in HBSS-1% MES buffer (0.5 mL). 1.5 mL of HBSS buffer-1% Hepes are added at basolateral level.

The LY transport is evaluated as the passage from the apical to the basolateral compartment after a defined incubation period of 1h at 37°C.

The reading is performed in the spectrofluorometer (TECAN) with 428 nm excitation and 535 nm emission. The fluorescence measurement (RFU) is performed at the apical and at the basolateral levels and flow and permeability of the marker are calculated according to the following formulas:

$$\text{Flow LY} = (\text{RFU BL} / \text{AP RFU}) \times 100$$

$$\text{Apparent permeability (PAPP, cm / sec)} =$$

$$(\text{BL Concentration} / \text{Concentration AP}) \times (\text{Volume BL} / \text{area} \times \text{time})$$

To derive the concentration of LY in the basolateral compartment, a standard curve is used which is obtained from different concentrations of LY in the 96-well plate (100 $\mu$l in triplicate):

LY: 0-6-12$\mu$M (low concentrations of standard)

LY: 0 - 6.12.25-100$\mu$M (high concentrations of standard)

In an intact monolayer, the flow of LY is less than 10% and the apparent permeability coefficient (Papp) is less than $2.3 \times 10^{-6}$ cm / sec (internal controls).

4.4 INTESTINAL PASS TEST

4.4.1 Principle of the method

The Caco-2 cells grown as a monolayer on a permeable artificial support reproduce the intestinal epithelial layer (monolayer of cells that are polarized and coupled by tight junctions). The flow through the monolayer allows to establish the permeability of a substance.

4.4.2 Procedure

After 21 days of growth on insert, the differentiated cells show an apical side, corresponding to the intestinal lumen in vivo, and one that corresponds to the basolateral compartment of the blood and lymphatic circulation of the organism.

The culture medium is aspirated from all cell monolayers before the experiments of permeability. All cell monolayers are washed once with HBSS solution (Hank's Balanced Salt Solution) and left to equilibrate at 37°C for 20 min.

The test substance (composition A) is prepared extemporaneously in 1X HBSS containing 1% MES (pH 6.5 to reproduce the low duodenal acidity) at 0.25% w/w concentration of Composition A. The test solution is added to the monolayer from the donor side (0.5 mL in triplicate), i.e. from the apical region. In parallel, the same amount of substance is divided into equal aliquots and represents the apical sample at time = 0 minutes (0'AP). At the receiving side of the monolayer 1.2 mL of 1X HBSS-Hepes 1% (pH 7.4) are added. The so treated plates are placed in an incubator at 37°C for a defined period of incubation (15-30-60 minutes).

In correspondence of each timepoint of the defined kinetics, samples are collected from the receiving side of the monolayers (0.6 mL). The studies are conducted within 60 min. Samples are taken at 15, 30, 60 minutes (defined as basolateral-BL). The volumes of the basolateral compartment are maintained constant, restoring the 1X HBSS after each sampling. At the end of treatments, the amount of substance remaining in the apical portion (T = 60'AP) is recovered and the cells are solubilized in 0.5 mL distilled water, re-suspended and preserved at 4°C for analysis and for determination of the mass balance.

The analysis of the amount of curcumin is made by HPLC-UV.

The apparent permeability coefficients Papp (cm / s) are determined by the following formula:

$$Papp = dQ / (dt \times A \times C0)$$

wherein:

- DQ / dt is the slope of the curcumin permeability profile in time (seconds) through the monolayer of Caco-2 cells (transferred mass per time unit)
- A is the surface area of the insert (1 cm$^2$)
- C0 is the concentration of the initial test of the donor

With the results obtained from the HPLC analysis, the calculation of the mass balance is performed as follows:

$$\% \text{ TOTAL MASS BALANCE} = (C\ 60'AP * 0.5\ ml * 100) / (Cd * 0.5\ ml + C\ 60'BL + C\ pellet * 0.5\ ml)$$

- C60' AP: final concentration of curcumin found in the apical compartment after 60 minutes
- C60' BL: concentration of curcumin found in the basolateral compartment after 60 minutes
- Cd: Initial concentration (donor) applied at the top level that takes into account the dilution factor within the relevant time interval.

4.4.3 Method of calculation

Using the mathematical formulas described by Arturrson the relationships are calculated between concentrations of substances in the basolateral (receiver) and in the apical (donor) compartments at each sampling time.

The values of "cumulative fraction Transported drug" (cm) are calculated from the experimentally found concentration data at basolateral level (Tavelin S. "Application of epithelial cell culture in studies of drug transport" Methods in Molecular Biology vol. 188 "Epithelial cell culture protocols"; Le Ferrec E. "In vitro models of the intestinal barrier" ECVAM workshop 46. ATLA 2001, 29, 649-668).

The outcome of the quantified substance in each compartment is shown in the following table 1:

Table 1

| COMPARTMENT | Outcome |
|---|---|
| Apical | NOT PENETRATED |
| Cellular Homogenate | Internalized |
| Basolateral | PENETRATED |

The cell homogenate and the basolateral compartment contain the curcumin fraction that is absorbed by passive transport.

5. Statistical analysis

No specific statistical analysis is carried out, with the exception of the calculation of standard deviations.

6. Results

6.1 Assessment of the toxicity of the core composition of the invention (Composition A) at three w/w concentrations - MTT TEST

Table 2

| Sample | Control (CN) | Composition A (0.25% w/w) | Composition A suspension (0.5% w/w) | Composition A suspension (1% w/w) |
|---|---|---|---|---|
| % of cell vitality | 100 | 36.892 | 35.812 | 32.161 |

MTT test after 2 hours of incubation with the core composition of the invention (Composition A) at three different w/w concentrations: 0.25% /0.5% / 1%.
The cytotoxicity of Composition A is tested at three different concentrations for 2h treatment: in all three (high) concentrations the product shows a cytotoxic effect that is not dose-dependent.
Since the intestinal uptake is expected to be over within 1h, it is decided to treat the cells for only 1h at 4 lower concentrations (Table 3). Also in this case, vitality values are found that are borderline, but higher than those for 2h and, in all cases, higher than 50%.

Table 3

| Sample | Control (CN) | Comp. A (0.025% w/w) | Comp. A (0.05% w/w) | Comp. A (0.1% w/w) | Comp. A (0.25% w/w) |
|---|---|---|---|---|---|
| % of cell vitality | 100 | 59.048 | 54.392 | 53.091 | 50.485 |

MTT test after 1 hour of incubation with COMPOSITION A at four different concentrations: 0.025% / 0.05% / 0.1% / 0.25%.
Based on these data, the highest non-cytotoxic concentration to be used for evaluating the intestinal passage of Composition A is 0.25%.
The theoretical concentration of curcumin inside the granules tested at 0.25% corresponds to 0.052% w/w = 1.404 mM.

6.2 Paracellular passage: evaluation of toxicity in tight junctions via Ly and TEER

All inserts prepared for the study presented initial values of TEER of about 200 $\Omega * cm^2$ (Table 4), that are valid to evaluate the toxicity of the core composition of the invention (COMPOSITION A) 0.25% by LY.
The inserts are treated for 1h with COMPOSITION A / LY; the CN insert (Negative untreated control) and blank insert are treated with LY only.

Table 4

| | Control | Composition A |
|---|---|---|
| Initial TEER values ($\Omega * cm^2$) | 188.7 | 190.65 |
| TEER values after 1 h ($\Omega * cm^2$) | 159.15 | 170.4 |

Measurement of TEER after 1 hour of incubation with COMPOSITION A 0.25% w/w solution
No difference in the values of TEER is found after treatment with the core composition of the invention in comparison to the cells in HBSS-CN 1% MES.

The results confirm a neutral action at the level of the structure of tight junctions. In Table 5 are shown the values of the paracellular Lucifer Yellow flux measured after 1h treatment with COMPOSITION A 0.25%.

Table 5

|  | Control (CN) | Composition A | Acceptable values |
|---|---|---|---|
| LY Flux % | 1.246% ± 0.021 | 1.148% ± 0.016 | <10% |
| Papp | 1.129* $10^{-6}$ | 8.040* $10^{-7}$ | <2.3* $10^{-6}$ cm/sec |

The core composition of the invention at the tested concentration is confirmed as nontoxic to junctions (Lucifer Yellow flow <10% and Papp <2.3 * $10^{-6}$ cm / sec)

6.3 Intestinal passage

6.3.1 Permeability test for curcumin dosage

The results of the curcumin detection by HPLC-UV are summarized in Table 6.

In the literature it was shown that curcumin has a low bioavailability, due to the low water solubility, and a high metabolism (Wahlang B, YB Pawar, AK Bansal. "Identification of permeability-related hurdles in oral delivery of curcumin using the Caco-2 cell model" Eur J Pharm Biopharm. 2011 Feb; 77 (2) :275-82).

To assess the linearity and stability of curcumin, curcuma rhizome 95% is used as reference and tetrahydrofuran (THF) must be used as solvent in a 1:3 ratio with the aqueous solvent (containing 1% Citric acid), considering the very low solubility of curcuma.

The analytical method used for the standard is found to be precise and accurate for the concentrations used (LOQ 0.05 mg / L, LOD 0.017 mg / L) but it is not sufficiently accurate to detect curcumin in the presence of only the buffers required for the absorption study, which partly restrict the solubility of curcumin.

The analytical results show that less than the theoretical amount of curcumin present in the initial sample is present in the 0.25% w/w solution of the core composition of the invention (Composition A), as reported in the following table 6 showing a loss of approximately 23% in title.

This loss is due to the incomplete solubility of the curcumin-containing samples in the buffers needed for the study: the solution in fact appears very cloudy and with a tendency
to precipitation.

Table 6

|  | TURMERIC EXTRACT containing 95% Curcumin (w/w) | THEORETICAL QUANTITY OF CURCUMIN In the 0.25% solution (w/w) | QUANTITY OF CURCUMIN detected by HPLC-UV technique in the 0.25 solution |
|---|---|---|---|
| Concentrations | 21.93 g/100g | 0.052% = 1.404 mM | 0.04% mM = 1.080 mM |

The % concentrations of Curcumin detected in different compartments, assayed in triplicate (3 inserts) after treatment with the core composition according to the invention (COMPOSITION A) for 1h, are shown in the following table 7:

Table 7

| Curcumin | | | |
|---|---|---|---|
| Compartment | % | μM | % Recovered compared to 0'AP |
| Apical at 60 min | 1) 0.01<br>2) 0.02<br>3) 0.01 | 1) 270<br>2) 540<br>3) 270 | 1) 25%<br>2) 50%<br>3) 25% |
| Homogenate Cell at 60 min | 1) 0.01<br>2) 0.01<br>3) 0.02 | 1) 270<br>2) 270<br>3) 540 | 1) 25%<br>2) 25%<br>3) 50% |
| Basolateral | 1) 0.00075 | 1) 20.25 | 1) 1.875% |

(continued)

| Curcumin | | | |
|---|---|---|---|
| Compartment | % | μM | % Recovered compared to 0'AP |
| (15 min +30 min | 2) 0.00075 | 2) 20.25 | 2) 1.875% |
| +60 min) | 3) 0.00075 | 3) 20.25 | 3) 1.875% |
| TOTAL: | 1) 0.02075 | 1) 560.25 | 1) 51,875% |
| APEX + | 2) 0.03075 | 2) 830.25 | 2) 76.875% |
| basolateral + CELL homogenate | 3) 0.03075 | 3) 830.25 | 3) 76.875% |
| (Apical = difference between reading in the solution initially applied and residual after 60 min, basolateral and homogenate= reading at the specified times) | | | |

It is evident that the dosages of curcumin in the analyzed biological samples are significantly influenced by the poor solubility of curcumin.

In fact, the recovery values of curcumin in (52% -77%) show that a significant % of curcumin has not been quantified in the tested Experimental conditions, which does not allow a correct calculation of the final mass balance.

However, the analysis of the distribution in the different compartments, though underestimated due to poor solubility (for example: in insert 1 only 0.01% is quantified in the cellular homogenate and 0.01% in the apical portion at the end of the period of incubation, after initial application of 0.04% of an initial quantity of one substance), shows at 60 minutes an equivalent distribution of curcumin between the apical and the cellular homogenate, that confirm its internalization in the gut epithelium.

No passage of curcumin in the basolateral compartment is detectable.

7. CONCLUSIONS

The in vitro study of intestinal passage of curcumin-comprising Composition A is carried out in Caco-2 cells at the test concentration of 0.25% as defined on the basis of the values of the preliminary cytotoxicity tests.

The data of both the paracellular passage with lucifer yellow and the TEER measure show that curcumin granulate (core composition of the invention) does not induce toxicity at the level of the intercellular junctions.

In this study it is not possible to calculate the mass balance (Mass balance, MB), i.e. the percentage of curcumin recovered in the various compartments at the end of the intestinal passage, due to the incomplete solubility of the core composition of the invention in the buffers used for the study.

23% of the theoretical curcumin content is not detected in the solution, consequently this loss is also applicable to the analysis of the samples generated in the study.

The sum of the amounts of compound recovered in the apical and in the basolateral compartment is then calculated, together with the fraction associated with the cellular component (homogenate) compared to the actual initially applied amount (51,875% and 76.875% in inserts 1 and 2 respectively).

In the present study, curcumin, a lipophilic molecule that is prone to intracellular accumulation, demonstrates a low permeability with a Papp (A-B) of $8.040 * 10^{-7}$.

This is confirmed in the literature (Wahlang B, YB Pawar, AK Bansal. "Identification of permeability-related hurdles in oral delivery of curcumin using the Caco-2 cell model" Eur J Pharm Biopharm. 2011 Feb; 77 (2) 275-82). In fact, curcumin is not detected in the basolateral compartment.

However, the analysis of the distribution in the different compartments, though underestimated due to the poor solubility of curcumin in buffers, already shows at 60 minutes an equivalent distribution of curcumin between the apical compartment and the cell homogenate, confirming its internalization at intestinal epithelium level.

Because the absorption of curcumin in water, that forms an opaque solution containing particulate matter is regulated by two phases, i.e. disintegration/dissolution and absorption through the mucous membrane, the internalization of the granulate form is found to be more rapid than other forms of administration.

For curcumin, that is a lipophilic substance, the limiting factor to the internalization is in fact its solubility in aqueous solution: the molecular fraction that is made available can internalize and accumulate faster at the cell monolayer level.

In support of this behaviour, it has been shown in literature that, using lysed cells, 11.78% of curcumin is metabolized during transport and accumulates in the cells (through studies in kinetics of absorption and release of curcumin). The accumulated amount is 20%. This figure is comparable with the accumulation results observed in the cellular homogenate: 25% (in 2 inserts) and even 50% (in 1 insert).

Thus, the core composition according to the present incention provides a safe and more efficient method to deliver with high rate of absorption an otherwise poorly bioavailable active substance.

**Claims**

1. A composition comprising:

   a) a core comprising at least one active principle with low bioavailability, a polysorbate and a N-acetylcysteine-chitosan salt;
   b) an enteric coating layer enclosing said core a),
   wherein said N-acetylcysteine-chitosan salt comprised in a) is obtained by the protonation of the free amino group of chitosan by means of N-acetyl-cistein, behaving as an acid.

2. The composition according to claim 1 wherein the layer b) is an enteric film directly applied onto the core a).

3. The composition according to claim 1 or 2 wherein the at least one active principle with low availability is selected from trans-resveratrol, melatonin, omega-3 fatty acids, steroidal terpenes as boswellic acids, non steroidal terpenes, saponins, essential oils, polyphenols including curcumin, berberin and mixtures thereof.

4. The composition according to one of the preceding claims wherein the at least one active principle with low availability is curcumin or berberin.

5. The composition according to one of the preceding claims wherein the polysorbate is polysorbate 80.

6. The composition according to one of the preceding claims wherein the core a) further comprises an extract of *Citrus x paradisi* titrated to at least 40% by weight/total weight of the extract of *Citrus x paradisi* in bioflavonoids, and specifically titrated in Naringin and Naringenin, and/or an extract of *Piper nigrum* titrated in piperine to 95% by weight/total weight of the extract of Piper nigrum.

7. The composition according to one of the preceding claims wherein the coating b) comprises or consists of shellac.

8. The composition according to one of the preceding claims in the form of an enteric granule, wherein the core a) is enclosed in an enteric film b, or of a gastro-resistant capsule wherein said core a) is enclosed in an enteric coated hard or soft shell (layer b)).

9. A tablet comprising the composition according to one of the preceding claims, wherein said tablet further comprises, outer to layer b), a layer c) comprising at least one excipient suitable for tabletting.

10. The composition according to claim 9 wherein at least one excipient of the outer layer c) suitable for the compression is selected from starch, microcrystalline cellulose, magnesium stearate, calcium phosphate, talc, silica and mixtures thereof.

11. The composition according to one of the preceding claims for use as a medicament.

12. A dietary supplement or nutraceutical composition comprising the composition according to one of claims 1-9.

13. Method for the formation of the composition according to one of claims 1-10 comprising the steps of:

   i. preparing a solution or aqueous dispersion comprising at least a polysorbate in a concentration between 1 and 30% by weight/total weight of the solution or aqueous dispersion and a N-acetylcysteine-chitosan salt, wherein N-acetyl cysteine and chitosan are in a concentration between 0.01 and 3% by weight/total weight of the solution;
   ii. wet-granulating a composition comprising at least the active principle with low bioavailability with the solution or aqueous dispersion obtained in step i.;
   iii. drying to constant weight and sieving the granulate obtained in step ii
   iv. forming the core a) of the composition by compression of the solid obtained in step iii.;
   v. coating of the core a) obtained in step iv. with a gastro-resistant enteric layer.

**14.** The method according to claim 13, wherein the granulation of step ii. is performed by combining the solution or aqueous dispersion obtained in step i. and a composition comprising the active substance with low bioavailability, an extract of *Citrus x paradisi* titrated to at least 40% by weight/total weight of the extract of *Citrus x paradisi* in flavonoids and specifically titrated in Naringin and Naringenin and an extract of *Piper nigrum* titrated in piperine to 95% in weight/total weight of the extract of *Piper nigrum* and, if necessary, N-acetylcysteine and chitosan in such quantities as to obtain in a single dosage form an amount of chitosan between 1 and 150 mg, an amount ofN-acetylcysteine between 1 and 75 mg, an amount of extract of grapefruit (*Citrus x paradisi*) between 1 and 300 mg, and a quantity of extract of *Piper nigrum* between 1 and 30 mg.

**15.** The method according to claim 13 or 14, further comprising:

vi. adding to the filmed core obtained in step v. at least one excipient suitable for tabletting;
vii. tabletting the composition obtained in step vi., obtaining the composition in the form of a tablet according to claim 9.

## Patentansprüche

**1.** Zusammensetzung umfassend:

a) einen Kern, umfassend wenigstens einen Wirkstoff mit geringer Bioverfügbarkeit, ein Polysorbat und ein N-Acetylcystein-Chitosansalz;
b) eine enterische Überzugsschicht, die den Kern a) umschließt,
wobei das in a) enthaltene N-Acetylcystein-Chitosansalz durch Protonierung der freien Aminogruppe von Chitosan mittels sauer wirkendem N-Acetylcystein erhalten wird.

**2.** Zusammensetzung nach Anspruch 1, wobei die Schicht b) ein enterischer Film ist, der direkt auf den Kern a) aufgebracht wird.

**3.** Zusammensetzung nach Anspruch 1 oder 2, wobei wenigstens ein Wirkstoff mit geringer Verfügbarkeit ausgewählt ist aus Trans-Resveratrol, Melatonin, Omega-3-Fettsäuren, steroidalen Terpenen wie Boswelliasäuren, nichtsteroidalen Terpenen, Saponinen, ätherischen Ölen, Polyphenolen einschließlich Curcumin, Berberin sowie Gemischen davon.

**4.** Zusammensetzung nach einem der vorstehenden Ansprüche, wobei wenigstens ein Wirkstoff mit geringer Verfügbarkeit Curcumin oder Berberin ist.

**5.** Zusammensetzung nach einem der vorstehenden Ansprüche, wobei das Polysorbat Polysorbat 80 ist.

**6.** Zusammensetzung nach einem der vorstehenden Ansprüche, wobei der Kern a) ferner einen Auszug aus *Citrus x paradisi* umfasst, der auf wenigstens 40 Gew.-% bezogen auf das Gesamtgewicht des Auszugs aus *Citrus x paradisi* in Bioflavonoiden und insbesondere in Naringin und Naringenin titriert ist, und/oder einen Auszug aus *Piper nigrum,* der in Piperin auf 95 Gew.-% bezogen auf das Gesamtgewicht des Auszugs aus Piper nigrum titiriert ist.

**7.** Zusammensetzung nach einem der vorstehenden Ansprüche, wobei der Überzug b) Schellack umfasst oder daraus besteht.

**8.** Zusammensetzung nach einem der vorstehenden Ansprüche in Form eines enterischen Granulats, wobei der Kern a) von einem enterischen Film b umschlossen oder eine magenresistente Kapsel ist, wobei der Kern a) von einem enterisch überzogen Hart- oder Weichmantel (Schicht b)) umschlossen ist.

**9.** Tablette umfassend die Zusammensetzung nach einem der vorstehenden Ansprüche, wobei die Tablette ferner äußerlich der Schicht b) eine Schicht c) umfasst, die wenigstens einen zum Tablettieren geeigneten Hilfsstoff umfasst.

**10.** Zusammensetzung nach Anspruch 9, wobei wenigstens ein Hilfsstoff der zur Komprimierung geeigneten äußeren Schicht c) ausgewählt ist aus Stärke, mikrokristalliner Zellulose, Magnesiumstereat, Calciumphosphat, Talkum, Siliciumdioxid und Gemischen davon.

**11.** Zusammensetzung nach einem der vorstehenden Ansprüche zur Verwendung als Medikament.

**12.** Nahrungsergänzungsmittel oder nutrazeutische Zusammensetzung, umfassend die Zusammensetzung nach einem der Ansprüche 1 bis 9.

**13.** Verfahren zur Bildung der Zusammensetzung nach einem der Ansprüche von 1 bis 10, umfassend folgende Schritte:

i. Zubereiten einer Lösung oder wässrigen Dispersion, umfassend wenigstens ein Polysorbat in einer Konzentration zwischen 1 und 30 Gew.-% bezogen auf das Gesamtgewicht der Lösung oder wässrigen Dispersion und ein N-Acetylcystein-Chitosansalz, wobei N-Acetylcystein und Chitosan in einer Konzentration zwischen 0,01 und 3 Gew.-% bezogen auf das Gesamtgewicht der Lösung vorliegen;
ii. Nassgranulieren einer Zusammensetzung, umfassend wenigstens den Wirkstoff mit geringer Bioverfügbarkeit mit der in Schritt i. erhaltenen Lösung oder wässrigen Dispersion;
iii. Trocknen bis zur Gewichtskonstanz und Sieben des in Schritt ii. erhaltenen Granulats;
iv. Bilden des Kerns a) der Zusammensetzung durch Komprimieren des in Schritt iii. erhaltenen Feststoffs;
v. Beschichten des in Schritt iv. erhaltenen Kerns a) mit einer magenresistenten enterischen Schicht.

**14.** Verfahren nach Anspruch 13, wobei das Granulieren gemäß Schritt ii. durch Kombinieren der in Schritt i. erhaltenen Lösung oder wässrigen Dispersion mit einer Zusammensetzung ausgeführt wird, die den Aktivstoff mit geringer Bioverfügbarkeit, einem Auszug aus *Citrus x paradisi,* der auf wenigstens 40 Gew.-% bezogen auf das Gesamtgewicht des Auszugs aus *Citrus x paradisi* in Flavonoiden und insbesondere in Naringin und Naringenin titriert ist, und einem Auszug aus *Piper nigrum,* der in Piperin auf 95 Gew.-% bezogen auf das Gesamtgewicht des Auszugs aus *Piper nigrum* titriert ist, und, falls erforderlich, N-Acetylcystein und Chitosan in entsprechenden Mengen umfasst, um in einer Einzeldosis eine Menge an Chitosan zwischen 1 und 150 mg, eine Menge an N-Acetylcystein zwischen 1 und 75 mg, eine Menge an Grapefruit-(Citrus *x paradisi*)-Auszug zwischen 1 und 300 mg und eine Menge an *Piper nigrum*-Auszug zwischen 1 und 30 mg zu erhalten.

**15.** Verfahren nach Anspruch 13 oder 14, ferner umfassend:

vi. Zugeben von wenigstens einem zum Tablettieren geeigneten Hilfsstoff zu dem in Schritt v. erhaltenen überzogenen Kern;
vii. Tablettieren der in Schritt vi. erhaltenen Zusammensetzung, wodurch die Zusammensetzung in Form einer Tablette nach Anspruch 9 erhalten wird.

**Revendications**

**1.** Composition comprenant :

a) un noyau comprenant au moins un principe actif à faible biodisponibilité, un polysorbate et un sel de N-acétylcystéine-chitosane ;
b) une couche d'enrobage entérique enfermant ledit noyau a),
dans laquelle ledit sel de N-acétylcystéine-chitosane compris dans a) est obtenu par la protonation du groupe amino libre de chitosane au moyen de N-acétylcystéine, se comportant comme un acide.

**2.** Composition selon la revendication 1, dans laquelle la couche b) est un film entérique directement appliqué sur le noyau a).

**3.** Composition selon la revendication 1 ou 2, dans laquelle l'au moins un principe actif à faible disponibilité est sélectionné parmi le trans-resvératrol, la mélatonine, des acides gras oméga-3, des terpènes stéroïdiens comme acides boswelliques, des terpènes non stéroïdiens, des saponines, des huiles essentielles, des polyphénols comprenant de la curcumine, de la berbérine et leurs mélanges.

**4.** Composition selon une des revendications précédentes, dans laquelle l'au moins un principe actif à faible disponibilité est de la curcumine ou de la berbérine.

**5.** Composition selon une des revendications précédentes, dans laquelle le polysorbate est le polysorbate 80.

**6.** Composition selon une des revendications précédentes, dans laquelle le noyau a) comprend en outre un extrait de *Citrus x paradisi* titré à au moins 40% en poids/poids total de l'extrait de *Citrus x paradisi* en bio-flavonoïdes, et spécifiquement titré en Naringine et Naringénine, et/ou un extrait de *Piper nigrum* titré en pipérine à 95% en poids/poids total de l'extrait de *Piper nigrum.*

**7.** Composition selon une des revendications précédentes, dans laquelle l'enrobage b) comprend ou est constitué de gomme-laque.

**8.** Composition selon une des revendications précédentes sous la forme d'un granulé entérique, dans laquelle le noyau a) est enfermé dans un film entérique b, ou d'une capsule gastro-résistante, dans laquelle ledit noyau a) est enfermé dans une enveloppe dure ou molle enrobée entérique (couche b)).

**9.** Comprimé comprenant la composition selon une des revendications précédentes, dans laquelle ledit comprimé comprend en outre, à l'extérieur de la couche b), une couche c) comprenant au moins un excipient adapté pour la production de comprimés.

**10.** Composition selon la revendication 9, dans laquelle au moins un excipient de la couche extérieure c) adaptée pour la compression est sélectionné parmi l'amidon, la cellulose microcristalline, le stéarate de magnésium, le phosphate de calcium, le talc, la silice et leurs mélanges.

**11.** Composition selon une des revendications précédentes pour l'utilisation comme un médicament.

**12.** Complément alimentaire ou composition nutraceutique comprenant la composition selon une des revendications 1 à 9.

**13.** Procédé pour la formation de la composition selon une des revendications 1 à 10 comprenant les étapes suivantes :

i. la préparation d'une solution ou dispersion aqueuse comprenant au moins un polysorbate dans une concentration entre 1 et 30% en poids/poids total de la solution ou dispersion aqueuse et un sel de N-acétylcystéine-chitosane, dans laquelle la N-acétylcystéine et le chitosane sont dans une concentration entre 0,01 et 3% en poids/poids total de la solution;
ii. la granulation par voie humide d'une composition comprenant au moins le principe actif à faible biodisponibilité avec la solution ou dispersion aqueuse obtenue dans l'étape i. ;
iii. le séchage à un poids constant et le tamisage du granulat obtenu dans l'étape ii. ;
iv. la formation du noyau a) de la composition par compression du solide obtenu dans l'étape iii. ;
v. l'enrobage du noyau a) obtenu dans l'étape iv. avec une couche entérique gastro-résistante.

**14.** Procédé selon la revendication 13, dans laquelle la granulation de l'étape ii. est effectuée en combinant la solution ou dispersion aqueuse obtenue dans l'étape i. et une composition comprenant la substance active à faible biodisponibilité, un extrait de *Citrus x paradisi* titré à au moins 40% en poids/poids total de l'extrait de *Citrus x paradisi* en flavonoïdes et spécifiquement titré en Naringine et Naringénine et un extrait de *Piper nigrum* titré en pipérine à 95% en poids/poids total de l'extrait de *Piper nigrum* et, si nécessaire, de la N-acétylcystéine et du chitosane dans des quantités permettant d'obtenir sous une forme de dosage unique une quantité de chitosane entre 1 et 150 mg, une quantité de N-acétylcystéine entre 1 et 75 mg, une quantité d'extrait de pamplemousse (*Citrus x paradisi)* entre 1 et 300 mg, et une quantité d'extrait de *Piper nigrum* entre 1 et 30 mg.

**15.** Procédé selon la revendication 13 ou 14, comprenant en outre :

vi. l'ajout au noyau enrobé de film obtenu dans l'étape v. d'au moins un excipient adapté pour la production de comprimés ;
vii. la production en comprimés de la composition obtenue dans l'étape vi., en obtenant la composition sous la forme d'un comprimé selon la revendication 9.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IT 2008VE00055
- WO 2011161501 A
- KR 100750727

### Non-patent literature cited in the description

- **SCHMITZ, T. et al.** *Drug Deliv.,* 2008, vol. 15, 245
- **CHEN, H. et al.** *J. Liposome Res.,* 2011
- **SAKLOESAKUN, D. et al.** *Drug Dev. Ind. Pharm.,* 2011, vol. 37, 648
- **SCHMITZ, T . et al.** *Int. J. Pharm.,* 2008, vol. 347, 79
- **HOMBACH, J. et al.** *Eur. J. Pharm. Sci.,* 2008, vol. 33, 1-8
- **LE FERREC E.** *ECVAM workshop 46,* 2001
- **GARATE M.A. et al.** *J. Biol. Chem.,* 2000, vol. 275, 1671
- **ARTURSSON, P ; BORCHARDT R.T.** Intestinal drug adsorption and metabolism in cell cultures: Caco-2 and beyond. *Pharma. Res.,* 1997, vol. 14, 1655-1658
- **HIDALGO.** Characterization of the human colon carcinoma cell line (Caco-2) as a model for intestinal epithelial permeability. *Gastroenterology,* 1989, vol. 96, 736-749
- **LE FERREC E.** In vitro models of the intestinal barrier. *ECVAM workshop 46, ATLA,* 2001, vol. 29, 649-668
- **TAVELIN S.** Application of epithelial cell culture in studies of drug transport'' Methods in Molecular Biology. *Epithelial cell culture protocols,* vol. 188
- **LE FERREC E.** In vitro models of the intestinal barrier. *ECVAM workshop 46. ATLA,* 2001, vol. 29, 649-668
- **WAHLANG B ; YB PAWAR ; AK BANSAL.** Identification of permeability-related hurdles in oral delivery of curcumin using the Caco-2 cell model. *Eur J Pharm Biopharm,* February 2011, vol. 77 (2), 275-82
- **WAHLANG B ; YB PAWAR ; AK BANSAL.** Identification of permeability-related hurdles in oral delivery of curcumin using the Caco-2 cell model. *Eur J Pharm Biopharm.,* February 2011, vol. 77 (2), 275-82